# EUROPEAN PATENT APPLICATION

(11) **EP 4 835 914 A1**
(43) Date of publication of application: **04.11.2026**
(21) Application number: 24897071.7
(22) Date of filing: 30.09.2024
(51) Int. Cl.: H01B 1/06, C07C 69/21, C07C 233/05, C07C 255/04, C07C 311/48, C07D 323/00, C07F 1/02, H01G 11/56, H01M 10/052, H01M 10/054, H01M 10/0565

(54) **MOLECULAR CRYSTAL, SOLID ELECTROLYTE, AND POWER STORAGE DEVICE**

(30) Priority: 29.11.2023 JP 2023202026
(71) Applicant: Toagosei Co., Ltd., Minato-ku Tokyo 105-8419 (JP)
(72) Inventor: AOYAMA, Satoru, Tokyo 105-8419 (JP); TANAKA, Ryouto, Tokyo 105-8419 (JP); HIRAOKA, Hideki, Tokyo 105-8419 (JP)
(74) Representative: TBK
(86) International application number: PCT/JP2024/034908
(87) International publication number: WO 2025/115381

(57) **Abstract**

A molecular crystal includes: an organic molecule having a total of four or more atoms of at least one species selected from the group consisting of a nitrogen atom, an oxygen atom, a sulfur atom, and a phosphorus atom; and an alkali metal salt.

## Description

### Technical Field

### [Cross-Reference to Related Applications]

The present application claims a priority from Japanese Patent Application No. 2023-202026 filed on November 29, 2023, the entirety of the disclosure of which is incorporated herein by reference.

The present disclosure relates to a molecular crystal, a solid electrolyte and a power storage device.

### Background Art

A variety of devices, including secondary batteries such as a nickel-hydrogen secondary battery and a lithium ion secondary battery, and an electric double layer capacitor, have been practically used as power storage devices. Among such secondary batteries, a lithium ion secondary battery finds a wide range of uses by virtue of high energy density and high battery capacity. In recent years, attention has been attracted to post-lithium ion secondary batteries employing an element instead of lithium (i.e., a rare metal), including a sodium ion secondary battery, a potassium ion secondary battery, and a magnesium ion secondary battery.

A lithium ion secondary battery, which is widely used as a power storage device, is a secondary battery including a negative electrode, a positive electrode, and an electrolyte, in which charge/discharge is achieved through transfer of lithium ions between the two electrodes by the mediation of the electrolyte. Conventionally, an organic electrolytic solution is mainly employed as an electrolyte. In contrast, in recent years, there has been proposed a solid or gel-form electrolyte employing an inorganic or organic material instead of such an organic electrolytic solution. The electrolyte has been proposed as a technique for overcoming concerns including liquid leakage of electrolytic solution and short circuit due to excessive charge and discharge in the battery.

The organic solid electrolyte is advantageous for appropriate flexibility intrinsic to organic material and high formability, to thereby attain excellent adhesion to an electrode. However, as compared with, for example, an inorganic solid electrolyte, the ionic conductivity is generally low. Thus, in order to enhance practicability, various studies have been conducted for the development of new materials for forming an organic solid electrolyte (see, for example, Non-Patent Document 1). Non-Patent Document 1 discloses that a crystalline organic solid electrolyte is formed from a soft solid crystal composed of lithium chloride and isoquinoline.

### Prior art Documents

### Non-Patent Document

Non-Patent Document 1: Ionics, Vol. 24, p. 343-349 (2018)

### Summary of the Invention

### Problems to be Solved by the Invention

For example, solid electrolytes used in automotive applications for power storage devices require high heat resistance to prevent melting even under high temperatures (e.g., above 80°C) to maintain the state of the material as a solid electrolyte (i.e., solid state) under various environmental conditions. However, crystalline organic solid electrolytes have insufficient heat resistance and may melt under high temperatures. In such cases, the electrolyte cannot maintain a solid state in the power storage device, and concerns about electrolyte leakage from the device and internal short circuit caused by excessive charge and discharge cannot be eliminated.

The present disclosure has been conceived in light of the above circumstances. Thus, an object of the present disclosure is to provide a molecular crystal that exhibits high ionic conductivity while maintaining a solid state under high temperatures. Another object is to provide a solid electrolyte that maintains a solid state under high temperatures while exhibiting high ionic conductivity, as well as a power storage device including this solid electrolyte.

### Means for Solving the Problems

The present inventors have conducted extensive studies to attain the aforementioned objects, and have found that a molecular crystal containing a specific organic molecule and an alkali metal salt as a constitutional unit in a crystal lattice exhibits high ionic conductivity while maintaining a solid state under high temperatures. According to the present disclosure, the following molecular crystal, solid electrolyte, and a power storage device are provided.

[1] A molecular crystal including: an organic molecule having a total of four or more atoms of at least one species selected from the group consisting of a nitrogen atom, an oxygen atom, a sulfur atom, and a phosphorus atom; and an alkali metal salt.
[2] The molecular crystal of [1], in which the organic molecule has a total of four or more atoms of either or both of nitrogen and oxygen atoms.
[3] The molecular crystal of [1] or [2], in which the organic molecule comprises a compound having two or more amide groups.
[4] The molecular crystal of any of [1] to [3], in which the organic molecule comprises a compound having four or more oxygen atoms.
[5] The molecular crystal of [4], in which the compound having four or more oxygen atoms is a crown ether or a polyol ester.
[6] The molecular crystal of any of [1] to [5], in which an ionic conductivity at 25°C is 1.0 × 10⁻⁶ S/cm or higher.
[7] The molecular crystal of any of [1] to [6], in which the alkali metal salt contains an imide-type alkali metal salt.
[8] A solid electrolyte containing the molecular crystal of any one of [1] to [7].
[9] A power storage device including the solid electrolyte of [8].

### Advantageous Effects of the Invention

According to the present disclosure, a molecular crystal and a solid electrolyte that exhibit high ionic conductivity while maintaining a solid state at high temperatures can be obtained. In addition, by employing such a solid electrolyte of the present disclosure as an electrolyte of a power storage device as a secondary battery or a capacitor, a power storage device can be obtained that provides both safety by electrolyte solidification and ionic conductivity.

### Brief Description of the Drawings

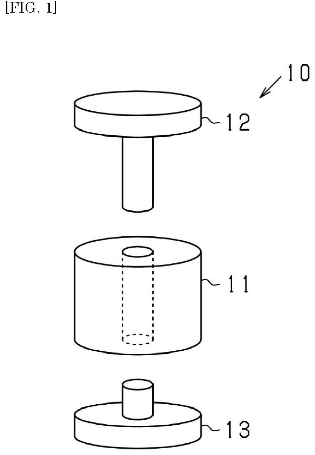

[Fig. 1] Fig. 1 is a schematic configuration of a press mold used in production of sample pellets.

### Modes for Carrying Out the Invention

The following describes in detail a molecular crystal, a solid electrolyte, and a power storage device of the present disclosure.

### <<Molecular crystal>>

A molecular crystal is a material in which constitutional units are regularly arranged in the crystal lattice. The molecular crystal of the present disclosure (hereinafter, also referred to as "the present molecular crystal") is a crystalline organic substance that contains, as a constitutional unit of the crystal, an organic molecule (hereinafter, also referred to as "organic molecule (M)") having a total of four or more atoms of at least one species selected from a group consisting of a nitrogen atom, an oxygen atom, a sulfur atom, and a phosphorus atom, and an alkali metal salt. The present molecular crystal possesses ion conduction paths based on regularly arranged constitutional units, whereby excellent ionic conductivity is provided.

In the present molecular crystal, no particular limitation is imposed on the ratio of the organic molecule (M) to the alkali metal salt. The ratio of the organic molecule (M) to the alkali metal salt in the molecular crystal may be from 0.1 to 10 mol of the organic molecule (M) with respect to 1 mol of the alkali metal salt. From the viewpoint of enhancing ionic conductivity, the ratio of the organic molecule (M) to the alkali metal salt in the molecular crystal is preferably from 0.2 to 5.0 mol of the organic molecule (M), more preferably 0.25 to 4.0 mol of the organic molecule (M), still more preferably 0.5 to 2.0 mol of the organic molecule (M), with respect to 1 mol of the alkali metal salt.

### <Organic molecule (M)>

The organic molecule (M) only needs to have, in one molecule, a total of four or more atoms of at least one species selected from the group consisting of a nitrogen atom, an oxygen atom, a sulfur atom, and a phosphorus atom (hereinafter also referred to as "specific heteroatom"). When the organic molecule forming the molecular crystal has a total of four or more specific heteroatoms in one molecule, the polarity of the organic molecule increases, by which volatilization and sublimation of the organic molecule during production of the molecular crystal are suppressed, and as a result, a molecular crystal with reduced variations in composition can conceivably be obtained. Also conceivably, more uniform ion conduction paths are formed in the molecular crystal by reducing variations in composition of the molecular crystal, thereby successfully yielding a molecular crystal exhibiting excellent ionic conductivity.

No particular limitation is imposed on the structure of the organic molecule (M). The organic molecule (M) may be a molecule having a chain structure, or may be a molecule having a cyclic structure. The molecular weight of the organic molecule (M) is, for example, 600 or lower. From the viewpoint of promoting crystallization of the organic molecule (M) and the alkali metal salt, the molecular weight of the organic molecule (M) is preferably 500 or lower, and more preferably 450 or lower. The lower limit of the molecular weight of the organic molecule (M) is, for example, 30 or higher, preferably 50 or higher, more preferably 80 or higher, and still more preferably 90 or higher.

The number of specific heteroatoms in one molecule of the organic molecule (M) is, from the viewpoint of evoking crystallization of the mixture of the organic molecule and the alkali metal salt to yield a molecular crystal exhibiting good ionic conductivity, preferably 4 to 30, more preferably 4 to 20, and still more preferably 4 to 10.

One embodiment of the organic molecule (M) includes a compound having, in one molecule, one or more functional groups containing a total of two or more of one or more species of specific heteroatoms (hereinafter also simply referred to as "polar groups"). Examples of the polar group include an amide group (-CO-NR¹R², -NR¹-CO-R³), a carboxyl group, an ester group (-COOR³), an acyloxy group (-OCOR³), a phosphate group (-O-P(=O)(-OR¹)₂), a phosphonic acid group (-P(=O)(-OR¹)₂), a sulfonic acid group, a sulfonyl group, and a sulfonyloxy group. Herein, R¹ and R² are each independently a hydrogen atom, an alkyl group having 1 to 6 carbon atoms, a cycloalkyl group, an aryl group, or an aralkyl group, and R³ is an alkyl group having 1 to 6 carbon atoms, a cycloalkyl group, an aryl group, or an aralkyl group. The two R¹s in one functional group may be the same or differ.

When a compound having a polar group containing a total of two or more specific heteroatoms is used as the organic molecule (M), the number of polar groups in one molecule of this compound is, from the viewpoint of promoting crystallization of the mixture with the alkali metal salt, preferably 2 to 10, more preferably 2 to 6, and still more preferably 2 to 4.

As the organic molecule (M), a compound having an ether bond, a thioether bond, or a tertiary amino group and having four or more specific heteroatoms in one molecule may also be used. Furthermore, as the organic molecule (M), a compound having the above-mentioned polar group, and an ether bond, a thioether bond, or a tertiary amino group, and having four or more specific heteroatoms in one molecule may also be used.

From the viewpoint of the ionic conductivity of the molecular crystal and the availability of the materials, as the organic molecule (M), a compound having in one molecule a total of four or more atoms of either one or both of nitrogen and oxygen atoms can be preferably used. In other words, as the organic molecule (M), one or more of a compound having four or more nitrogen atoms in one molecule, a compound having four or more oxygen atoms in one molecule, and a compound having nitrogen atoms and oxygen atoms and having in one molecule four or more nitrogen atoms and oxygen atoms in total can be preferably used. When a compound having in one molecule a total of four or more atoms of either one or both of nitrogen and oxygen atoms is used as the organic molecule (M), preferable specific examples of this compound include a compound having two or more amide groups, and a compound having four or more oxygen atoms.

A compound having two or more amide groups only needs to have in one molecule a total of two or more amide groups (-CO-NR¹R², -NR¹-CO-R³). When a compound having two or more amide groups is used as the organic molecule (M), the number of amide groups in one molecule of this compound is, from the viewpoint of promoting crystallization of the mixture of the organic molecule (M) and the alkali metal salt, preferably 2 to 10, more preferably 2 to 6, and still more preferably 2 to 4.

Compounds having four or more oxygen atoms include a crown ether, a compound having two or more ester groups (-COOR³), a compound having two or more acyloxy groups (-OCOR³), (i.e., polyol ester), a lactide, etc. Among these, a crown ether, a polyol ester, or a lactide is preferable. When a compound having four or more oxygen atoms is used as the organic molecule (M), the number of oxygen atoms in one molecule of this compound is, from the viewpoint of promoting crystallization of the mixture of the organic molecule (M) and the alkali metal salt, preferably 4 to 20, more preferably 4 to 14, and still more preferably 4 to 10.

Specific examples of the organic molecule (M) include: malonamide; succinamide; glutaramide; adipamide; N,N,N',N'-tetramethylmalonamide; N,N,N',N'-tetraethylmalonamide; N,N,N',N'-tetramethylsuccinamide; terephthalamide; N,N'-diacetylethylenediamine; N,N,N',N'-ethylenediamine tetrakis (methylenephosphonic acid); 12-crown-4 ether; 15-crown-5 ether; 18-crown-6 ether; pentaerythritol tetraacetate; glycolide; 1,4,8,11-tetrathiacyclotetradecane; 1,3-propanesultone; 1,4-butanesultone; tetraethyl ethylenediphosphonate; and N,N,N',N'-ethylenediamine tetrakis (methylenephosphonic acid). The organic molecule (M) may be used singly or in combination of two or more species, as long as crystallization is evoked together with an alkali metal salt.

### <Alkali metal salt>

No particular limitation is imposed on the alkali metal salt, so long as the salt generates an alkali metal ion. Examples of the alkali metal salt include a lithium salt, a sodium salt, and a potassium salt.

Specific examples of the alkali metal salt include lithium salts such as Li₂CO₃, LiBr, LiCl, LiI, LiSCN, LiBF₄, LiAsF₆, LiClO₄, CH₃COOLi, CF3COOLi, LiCF₃SO₃, LiPF₆, LiC(CF₃SO₂)₃, lithium bis(fluorosulfonyl)imide (Li⁺(FSO₂)₂N⁻), lithium bis(trifluoromethanesulfonyl)imide (Li+(CF₃SO₂)2N⁻), and lithium(fluorosulfonyl)(trifluoromethanesulfonyl)imide; and salts of an anion of the above lithium salts and an alkali metal other than lithium (e.g., sodium or potassium). From the viewpoint of high ion dissociation performance and achieving higher ionic conductivity of the present molecular crystal, among others, a lithium salt or a sodium salt is preferable, with a lithium salt being more preferable.

From the viewpoint of achieving higher ionic conductivity of the present molecular crystal, the alkali metal salt forming the present molecular crystal preferably includes an imide-type alkali metal salt. From the viewpoint of high ion dissociation performance, imide-type lithium salts are preferable among other imide-type alkali metal salts, and particularly among imide-type lithium salts, lithium bis(fluorosulfonyl)imide, lithium bis(trifluoromethanesulfonyl)imide, or lithium(fluorosulfonyl)(trifluoromethanesulfonyl)imide is preferable, with lithium bis(fluorosulfonyl)imide or lithium bis(trifluoromethanesulfonyl)imide being more preferable, lithium bis(fluorosulfonyl)imide being still more preferable.

The molecular weight of the alkali metal salt is, for example, 500 or lower, preferably 400 or lower, more preferably 350 or lower, still more preferably 300 or lower. The lower limit of the molecular weight of the alkali metal salt is, for example, 20 or higher, preferably 50 or higher, more preferably 100 or higher, still more preferably 150 or higher. The alkali metal salt may be used singly or in combination of two or more species.

The melting point of the alkali metal salt under atmospheric pressure is, for example, 60°C or higher, preferably 70°C or higher, and more preferably 80°C or higher. No particular limitation is imposed on the upper limit of the melting point of the alkali metal salt, and the melting point is, for example, 300°C or lower, and may be 250°C or lower.

It should be noted that as long as the effects of the present disclosure are not impaired, the present molecular crystal may further include a constitutional unit differing from those in the organic molecule (M), and the alkali metal salt (for example, an organic molecule with three or fewer specific heteroatoms). However, from the viewpoint of ionic conductivity and ease of production of the present molecular crystal, the present molecular crystal is preferably a molecular crystal constituted from the organic molecule (M) and the alkali metal salt.

The present molecular crystal exhibits high ionic conductivity not only at room temperature (25°C) but also at temperatures higher than room temperature (for example, 80°C or higher). Specifically, a solid electrolyte sample having a thickness of about 200 µm (specifically, 200 ± 20 µm) preferably exhibits an ionic conductivity, as measured through the AC impedance method at 25°C, of 1.0 × 10⁻⁶ S/cm or higher. From the viewpoint of yielding a high-performance power storage device, the ionic conductivity, as measured under the same conditions, is more preferably 1.5 × 10⁻⁶ S/cm or higher, still more preferably 2.0 × 10⁻⁶ S/cm or higher. Details of the measurement of ionic conductivity are in accordance with the method described in the below-mentioned Examples.

Also, the ionic conductivity of a solid electrolyte sample having a thickness of about 200 µm (specifically, 200 ± 20 µm), as measured through the AC impedance method at 80°C, is preferably 1.0 × 10⁻⁶ S/cm or higher, more preferably 5.0 × 10⁻⁵ S/cm or higher, still more preferably 1.0 × 10⁻⁴ S/cm or higher.

The melting point of the present molecular crystal is preferably 80°C or higher. The melting point of the present molecular crystal being 80°C or higher is preferable in that a molecular crystal with excellent heat resistance can be obtained. From the viewpoint of enabling application in uses where heat resistance is required, the melting point of the present molecular crystal is more preferably 82°C or higher, still more preferably 85°C or higher, even more preferably 87°C or higher, and yet more preferably 90°C or higher. No particular limitation is imposed on the melting point of the present molecular crystal. The melting point of the present molecular crystal is, for example, 300°C or lower. From the viewpoint of achieving higher ionic conductivity of the present molecular crystal, the melting point of the present molecular crystal is preferably 250°C or lower, more preferably 200°C or lower, and still more preferably 150°C or lower. In the present specification, the melting point of the molecular crystal is a value measured under atmospheric pressure and is a value as determined through differential scanning calorimetry (DSC). Details of the measurement are in accordance with the method described in the below-mentioned Examples.

### <Method for producing molecular crystal>

The present molecular crystal can be produced using the organic molecule (M) and the alkali metal salt as raw material. The method for producing the present molecular crystal is not particularly limited. The present molecular crystal can be produced, for example, by: a method (hereinafter also referred to as a "first producing method") including a step of mixing an organic molecule (M) with an alkali metal salt while applying mechanical energy (hereinafter also referred to as "mechanical mixing step"); and a method (hereinafter also referred to as "second producing method") including a step of dissolving an organic molecule (M) and an alkali metal salt in a solvent (hereinafter referred to as "dissolving step"), and a step of removing the solvent from a solution in which the organic molecule (M) and the alkali metal salts are dissolved (hereinafter referred to as "removing step").

### (First producing method)

### · Mechanical mixing step

In the mechanical mixing step, mechanical energy is applied to a target material containing the organic molecule (M) and the alkali metal salt by means of impact, shear, compression, friction, and the like, whereby mixing of the organic molecule (M) with the alkali metal salt (hereinafter also referred to as "mechanical mixing") is carried out. Thereby, a change in physicochemical property is preferably induced in the organic molecule (M) or the alkali metal salt or both through mechanical mixing.

No particular limitation is imposed on the ratio of the organic molecule (M) to the alkali metal salt used, and the ratio may be set in accordance with the type and the like of the organic molecule (M) and the alkali metal salt. The ratio of the organic molecule (M) to the alkali metal salt used may be adjusted to, for example 0.1 to 10 mol of the organic molecule (M) with respect to 1 mol of the alkali metal salt. The ratio of the organic molecule (M) to the alkali metal salt used is preferably adjusted to 0.2 to 5.0 mol of the organic molecule (M), more preferably 0.25 to 4.0 mol of the organic molecule (M), still more preferably 0.5 to 2.0 mol of the organic molecule (M), with respect to 1 mol of the alkali metal salt, from the viewpoint of the capability to obtain a molecular crystal exhibiting high ionic conductivity.

No particular limitation is imposed on the method for mixing the organic molecule (M) and the alkali metal salt under application of mechanical energy. In the mechanical mixing step, mixing of the organic molecule (M) with the alkali metal salt may be conducted by means of various apparatuses, such as a ball mill, a bead mill, a blender, a homogenizer, a stamp mill, a homo-mixer, and a dispersion mixer. When production of the molecular crystal is conducted on a small scale, the organic molecule (M) may also be mixed mechanically with the alkali metal salt by means of a mortar and a pestle. The mechanical mixing of the organic molecule (M) with the alkali metal salt may be conducted in a dry format or a wet format. In addition, the mechanical mixing of the organic molecule (M) with the alkali metal salt may be conducted at ambient temperature or low temperature. For example, when the melting point of the organic molecule (M) is lower than room temperature, the organic molecule (M) in the liquid state may be mechanically mixed with the alkali metal salt. Moreover, the organic molecule in a solid state at a temperature lower than the melting point of the organic molecule (M) may be mechanically mixed with the alkali metal salt.

Notably, the mechanical mixing of the organic molecule (M) with the alkali metal salt may be conducted under heating. However, in order to suppress volatilization or sublimation of the organic molecule (M), heating in the mechanical mixing step is preferably conducted at a temperature lower than the heating temperature in a heating step optionally employed after the mechanical mixing step. Specifically, in the mechanical mixing step, the mechanical mixing of the organic molecule (M) with the alkali metal salt is conducted at a temperature of 50°C or lower, for example. The temperature of mechanically mixing the organic molecule (M) with the alkali metal salt is preferably 40°C or lower, more preferably 35°C or lower. The time for the mechanical mixing, which depends on the amount, type, etc. of the organic molecule (M) or the alkali metal salt, is, for example, 1 to 60 minutes and may be 1 to 30 minutes.

When producing a molecular crystal by the first producing method, the first producing method further preferably includes a step of heating a mixture obtained by the mechanical mixing step (i.e., a mixture of the organic molecule (M) and the alkali metal salt) (hereinafter also referred to as a "heating step"). The heating step can attain a more homogeneous state of the organic molecule (M) and the alkali metal salt in the mixture, further enhancing the ionic conductivity of the molecular crystal. Notably, according to the method in combination of the mechanical mixing step and the heating step, intermolecular interaction is conceivably generated by mechanical mixing between alkali metal ions derived from the alkali metal salt and organic molecule (M), resulting in the capability of suppressed volatilization or sublimation of the organic molecule (M) during the heat treatment. This provides an advantage of easily obtaining a molecular crystal with a desired composition.

### · Heating step

In the heating step, the temperature and method for heating the mixture are not particularly limited. The temperature of heating the mixture may be appropriately set in accordance with the type and the like of the organic molecule (M) and the alkali metal salt serving as raw materials. From the viewpoint of attaining a more homogeneous state of the organic molecule (M) and the alkali metal salt in the mixture, the heating temperature is, for example, 40°C or higher, more preferably 50°C or higher, still more preferably 60°C or higher. No particular limitation is imposed on the heating time, as long as homogenization of the organic molecule (M) and the alkali metal salt in the mixture can be achieved. The heating time is, for example, 1 minute to 3 hours, preferably 3 minutes to 2 hours. The heat treatment is generally conducted under normal pressure. However, the heating treatment may be conducted under pressurized conditions or reduced pressure.

Preferably, heating of the mixture is conducted under agitation of the mixture, so as to attain further homogenization of the organic molecule (M) and the alkali metal salt. No particular limitation is imposed on the mixing method, and examples of the mixing means include a magnetic stirrer, an agitation rod, an agitator equipped with an agitation paddle, and an external circulation agitator. Alternatively, agitation may also be conducted via mechanical means that can provide greater shear force such as a homo-mixer, a dispersion mixer, or a homogenizer.

After completion of heating the mixture containing the organic molecule (M) and the alkali metal salt, the heated mixture can be crystallized by lowering the temperature of the heated mixture, to thereby yield a target molecular crystal. During lowering the temperature of the heated mixture, the mixture may be gradually cooled (e.g., slowly cooled at room temperature over 1 to 48 hours). Alternatively, the heated mixture may be quenched (e.g., placing the heated mixture in a thermostatic chamber controlled at a temperature lower than room temperature (e.g., 15°C or lower) for a short period of time). From the viewpoint of suitably promoting crystallization of the heated mixture, gradually lowering the temperature of the mixture is preferable. The product of the present producing method being a molecular crystal containing the organic molecule (M) and the alkali metal salt can be detected through powder X-ray diffraction measurement of the product. Details of the measurement are in accordance with the method described in the below-mentioned Examples.

### (Second producing method)

### · Dissolving step

If a melting point of the organic molecule (M) is high (e.g., 100°C or higher), it is preferable that the second producing method be employed as a method to produce the present molecular crystal. An organic solvent can be used preferably as the solvent used for dissolving the organic molecule (M) and the alkali metal salt. This organic solvent is preferably a solvent that can dissolve the organic molecule (M) and the alkali metal salt, and has a boiling point lower than the respective boiling points of the organic molecule (M) and the alkali metal salt, more preferably a solvent that has a relatively low dielectric constant. Examples of such an organic solvent include dimethyl carbonate, tetrahydrofuran, and ethyl acetate. Preferably, dissolving the organic molecule (M) and the alkali metal salt into the solvent is conducted under stirring, so as to attain homogenization of the organic molecule (M) and the alkali metal salt. The method of stirring is not particularly limited, but for example, the stirring method exemplified in the first producing method can be used appropriately.

### · Removing step

No particular limitation is imposed on the method of removing the solvent from the solution in which the organic molecule (M) and the alkali metal salt are dissolved, as long as the solvent can be removed from the solution. A heat treatment is preferable for removing the solvent from the viewpoint of easily and efficiently removing the solvent. The heat treatment is generally conducted under normal pressure. However, the heating treatment may be conducted under pressurized conditions or reduced pressure. When a solvent with a high boiling point is used in the dissolving step, it is preferable to conduct a heat treatment under reduced pressure to suppress decomposition of the organic molecule (M) and the alkali metal salt.

The method for producing the present molecular crystal is not limited to the above. For example, in the first producing method described above, the present molecular crystal may be produced by heating and mixing the organic molecule (M) and the alkali metal salt, without conducting the mechanical mixing.

### <<Solid electrolyte>>

The solid electrolyte of the present disclosure contains the present molecular crystal described above. The solid electrolyte of the present disclosure can be obtained by molding an electrolyte material containing the present molecular crystal into a desired shape. No particular limitation is imposed on the molding method, and there may be employed a known method such as extrusion molding, injection molding, press molding, cast molding, mold casting, and tape molding. Among these molding methods, a molded product is preferably obtained through press molding, from the viewpoint of reducing, to a maximum extent, the vacancy of the solid electrolyte to facilitate formation of ion conduction paths. No particular limitation is imposed on the shape of the molded product, and the shape may be chosen in accordance with the shape of the power storage device to which the molded product is applied. The molded product has a shape of, for example, a rectangular shape or a circular shape.

As long as the effects of the present disclosure are not impaired, the solid electrolyte of the present disclosure may further contain an additional component other than the molecular crystal of the present disclosure. Examples of the additional component include another molecular crystal differing from the present molecular crystal, a polymer solid electrolyte, a polymer gel electrolyte, inorganic electrolytes (e.g., a ceramic electrolyte and a glass electrolyte), and a binder.

From the viewpoint of obtaining a solid electrolyte exhibiting excellent ionic conductivity particularly under high temperatures, the solid electrolyte preferably contains the present molecular crystal in a relative amount, with respect to the entire amount of the solid electrolyte, of preferably 50 mass% or more, more preferably 70 mass% or more, still more preferably 80 mass% or more, even more preferably 90 mass% or more.

The solid electrolyte of the present disclosure exhibits high ionic conductivity, while maintaining a solid state, not only at room temperature, but also under high temperatures of 80°C or higher. Thus, by using the solid electrolyte of the present disclosure as the electrolyte of the power storage device, a power storage device that is applicable to uses expected to involve operation under high temperatures (for example, automotive applications), and that exhibits high ionic conductivity can be obtained.

### <<Power storage device>>

The power storage device of the present disclosure (hereinafter may also be referred to as "the present device") has the solid electrolyte of the present disclosure. Specific embodiments of the present device include a secondary battery and a capacitor. When the present device is a secondary battery, one embodiment thereof is an all-solid-state battery. From the viewpoint of achieving excellent ionic conductivity, a lithium ion secondary battery is preferred.

The all-solid-state lithium ion secondary battery, which is an embodiment of the present device, will be described. The lithium ion secondary battery is a stacked body having electrodes including a positive electrode and a negative electrode, and a solid electrolyte. The solid electrolyte is disposed between the positive electrode and the negative electrode, such that the solid electrolyte is in contact with the electrodes. No particular limitation is imposed on the material forming the positive electrode and the negative electrode, and the material may be appropriately selected from materials known as the electrode material for the lithium ion secondary battery. For example, the positive electrode current collector may be formed from a foil of a metal such as aluminum or stainless steel. The negative electrode current collector may be formed from a metal foil such as copper foil and lithium foil.

In the lithium ion secondary battery of the present disclosure, the solid electrolyte is formed from a molecular crystal containing the organic molecule (M) and the alkali metal salt. No particular limitation is imposed on the thickness of the solid electrolyte, and the thickness may be appropriately set in accordance with use of the secondary battery and the like. The thickness of the solid electrolyte is, for example, 5 to 500 µm.

No particular limitation is imposed on the method of producing the lithium ion secondary battery, and any known method may be appropriately employed in accordance with the structure of the target battery or the like. In one possible procedure, a molded product formed from the molecular crystal is annealed, and the thus-obtained solid electrolyte is sandwiched by a positive electrode and a negative electrode, to thereby fabricate a stacked body including a positive electrode, a solid electrolyte, and a negative electrode. In an alternative procedure, an electrolyte material containing the molecular crystal is sandwiched by a positive electrode and a negative electrode, and the product is placed in a container. The container is subjected to annealing, to thereby fabricate a stacked body including a positive electrode, a solid electrolyte, and a negative electrode. The stacked body including a positive electrode, a solid electrolyte, and a negative electrode is typically housed in a case for use as a secondary battery.

Notably, the present device is not limited to the aforementioned configuration in which lithium ions serve as the ion conduction carrier, and may be a secondary battery employing, as a carrier, another ion species such as sodium ions. Alternatively, the present device may also be a capacitor. In one embodiment, the capacitor has a positive electrode, a negative electrode, and a solid electrolyte, in which the solid electrolyte is disposed between the positive electrode and the negative electrode, such that the solid electrolyte is in contact with the electrodes.

The power storage device employing the present solid electrolyte may find a variety of uses. Examples of the possible use thereof include power sources for: mobile devices such as mobile phones, personal computers, smart phones, game machines, and wearable terminals; mobile bodies such as electric vehicles, hybrid vehicles, robots, and drones; electronic/electric appliances such as digital cameras, video cameras, music players, electric power tools, and domestic electric products; etc.

### Examples

The present disclosure will be described more specifically by way of examples. It will be appreciated that the present disclosure should not be construed as limiting the disclosure thereto. Unless otherwise specified, the units "part(s)" and "%" refer to "part(s) by mass" and " mass%, " respectively.

### <<Production and evaluation of molecular crystal>>

### [Example 1]

### 1. Production of molecular crystal

In a glove box with an argon atmosphere, 38 parts of succinamide (product of Tokyo Chemical Industry Co., Ltd.) serving as an organic molecule and 62 parts of lithium bis(fluorosulfonyl)imide (product of Kanto Chemical Co., Inc.) serving as an alkali metal salt were weighed so as to attain a molar ratio of 1:1, and mechanically mixed together by means of a mortar at room temperature (25°C) for 15 minutes, to thereby yield a mixture. Next, the mixture obtained via mechanical mixing of raw materials was transferred to a vial, and stirred for 10 minutes by means of a magnetic stirrer controlled at 80°C. Thereafter, the mixture was allowed to stand to room temperature, to thereby yield a white solid.

The melting point of the white solid was measured by means of a differential scanning calorimeter (product of TA Instruments, DSC250, measurement atmosphere: nitrogen) at a temperature elevation rate of 10°C/minute. In the measurement, a significant endothermic peak (melting point) was observed at 97°C.

### 2. Evaluation

### (Determination of crystallinity through powder X-ray diffraction measurement)

The thus-obtained white solid was subjected to powder X-ray diffraction measurement by means of an X-ray diffractometer (D8 ADVANCE, product of Bruker AXS). In diffractometry, CuKα was employed as an X-ray source, and the applied voltage and current were adjusted to 40 kV and 40 mA, respectively. The measurement range, scanning rate, and step angle were adjusted to 2θ=5-55 degrees, 2.3 degree/minute, and 0.02 degrees, respectively. The case where a sharp peak was observed in an X-ray diffraction spectrum within a measurement range of angle indicates that the measurement sample (solid) has crystallinity. Thus, in the case where a sharp peak was observed within a measurement angle range, and an X-ray diffraction spectrum pattern different from that of each of the organic molecule and the alkali metal salt used as raw materials was observed, formation of a molecular crystal composed of the organic molecule and the alkali metal salt was confirmed.

The white solid obtained through the aforementioned procedure was subjected to powder X-ray diffraction measurement. A sharp peak was observed within the measurement angle range, and an X-ray diffraction spectrum different from those of the raw materials was observed. Consequently, the obtained white solid was found to have crystallinity, in other words, the white solid was confirmed to be a molecular crystal composed of the organic molecule and the alkali metal salt.

### (Ionic conductivity measurement)

By employing a press mold 10 composed of a die set 11, a top punch 12, and a bottom punch 13 (see Fig. 1), a sample pellet was fabricated in a glove box with an argon atmosphere. Firstly, a white solid (molecular crystal) (0.0200 g) was charged into the die set 11, and the die set was mounted on the bottom punch 13. The top punch 12 was placed on the die set 11. The white solid was pressed at 10 MPa for 1 minute by means of a hydraulic press machine, to thereby prepare a circular-shaped sample pellet with a diameter of 1 cm. Then, in a glove box with an argon atmosphere, the sample pellet was enclosed in an all-solid-state battery evaluation cell (KP-SolidCell, product of Hohsen Corp.).

By use of the all-solid-state battery evaluation cell in which the sample pellet was enclosed, ionic conductivity of the sample was measured in air. In the ionic conductivity measurement, while the all-solid-state battery evaluation cell in which the sample pellet was enclosed was thermally treated in a thermostatic chamber at 80°C (corresponding to thermal treatment temperature specified in Table 1), electric resistance was measured by AC impedance measurement. When the resistance value became constant, the thermal treatment was terminated. Subsequently, electric resistanfce at 25°C and 80°C was measured by AC impedance measurement. By use of the measurements of resistance, ionic conductivity (σ) was calculated by the following numerical expression (1). Notably, measurement of AC impedance for calculating the ionic conductivity was conducted after maintenance of the cell at the measurement temperature for 2 hours in a thermostatic chamber. $σ = L / \left(R\times S\right)$ (1) (wherein, in numerical expression (1), σ represents an ionic conductivity (unit: S/cm); R represents a resistance (unit: Ω); S represents a cross-sectional area employed in measurement of molecular crystal (unit: cm²); and L represents an inter-electrode distance (unit: cm).)

The ionic conductivity measurements of the obtained molecular crystal at 25°C and 80°C were 8.1 × 10⁻⁴ S/cm and 1.8 × 10⁻³ S/cm, respectively. The obtained molecular crystals maintained a solid state even at a temperature of 80°C.

### [Examples 2 to 5]

The procedure of Example 1 was repeated, except that the types and amounts of raw materials, and thermal treatment temperatures in ionic conductivity measurement were modified as specified in Table 1, to thereby yield molecular crystals. Each molecular crystal was subjected to differential scanning calorimetry, and ionic conductivity measurement at 25°C and 80°C. The results are shown in Table 1. Each molecular crystal maintained a solid state even at a temperature of 80°C.

### [Comparative Example 1]

In a glove box with an argon atmosphere, 30 parts of succinonitrile (product of Tokyo Chemical Industry Co., Ltd.) serving as an organic molecule and 70 parts of lithiumbis(trifluoromethanesulfonyl)imide (product of FUJIFILM Wako Pure Chemical Corporation, Product name: Lithium Bis(trifluoromethanesulfonyl)imide) serving as an alkali metal salt were weighed so as to attain a molar ratio of 3:2, and mechanically mixed together by means of a mortar at room temperature (25°C), to thereby yield a white solid (molecular crystal). The melting point of the molecular crystal obtained via mechanical mixing of raw materials obtained via mechanical mixing of raw materials was measured through differential scanning calorimetry. The melting point was 55°C. In addition, the ionic conductivity of the molecular crystal obtained via mechanical mixing of raw materials was measured at 25°C and 80°C in the same manner as in Example 1, except that the thermal treatment temperature for ionic conductivity measurement was at 40°C. The results are shown in Table 1. In this Comparative Example 1, the molecular crystal melted at 80°C, and the performance thereof as a solid electrolyte could not be evaluated.

**[Table 1]**

| Examples/Comparative Examples No. | | | | Example 1 | Example 2 | Example 3 | Example 4 | Example 5 | Comparative Example 1 |
|---|---|---|---|---|---|---|---|---|---|
| Molecular Crystal | Organic molecule (part) | | SA | 38 | | | | | |
| | | | MA | | 21 | | | | |
| | | | 12-crown-4 | | | 38 | | | |
| | | | PETAc4 | | | | 51 | | |
| | | | GL | | | | | 29 | |
| | | | SN | | | | | | 30 |
| | Alkali metal salt (part) | | LiFSI | 62 | 79 | | | 71 | |
| | | | LiTFSI | | | 62 | 49 | | 70 |
| | Organic molecule / Alkali metal salt (molar ratio) | | | 1.0 | 0.5 | 1.0 | 1.0 | 0.7 | 1.5 |
| Results of evaluation | Melting point (°C) | | | 97 | 89 | 86 | 148 | 89 | 55 |
| | ionic conductivity measurement | Thermal treatment temperature (°C) | | 80 | 70 | 60 | 100 | 60 | 40 |
| | | 25°C (S/cm) | | 8.1.E-04 | 1.7.E-04 | 1.0.E-05 | 1.0.E-07 | 1.2.E-04 | 2.3.E-05 |
| | | 80°C (S/cm) | | 1.8.E-03 | 5.5.E-04 | 8.6.E-05 | 3.6.E-06 | 2.7.E-04 | - |

Details of the compounds listed in Table 1 are as follows.
· SA: succinamide (product of Tokyo Chemical Industry Co., Ltd.), number of specific heteroatoms: 4 (two nitrogen atoms and two oxygen atoms)
· MA: malonamide (product of Tokyo Chemical Industry Co., Ltd.), number of specific heteroatoms: 4 (two nitrogen atoms, two oxygen atoms) 12-crown-4: 12-crown-4 ether (product of Tokyo Chemical Industry Co., Ltd.), number of specific heteroatoms: 4 (four oxygen atoms)
· PETAc4: pentaerythritol tetraacetate (product of Tokyo Chemical Industry Co., Ltd.), number of specific heteroatoms: 8 (eight oxygen atoms)
   GL: glycolide (product of Tokyo Chemical Industry Co., Ltd.), number of specific heteroatoms: 4 (four oxygen atoms)
· SN: succinonitrile (product of Tokyo Chemical Industry Co., Ltd.), number of specific heteroatoms: 2 (two nitrogen atoms)
· LiFSI: lithium bis(fluorosulfonyl)imide (product of Kanto Chemical Co., Inc.)
· LiTFSI: lithium bis(trifluoromethanesulfonyl)imide (product of FUJIFILM Wako Pure Chemical Corporation, product name: Lithium Bis(trifluoromethanesulfonyl)imide)

### <<Results of evaluation>>

As is clear from the results of Example 1 to 5, a molecular crystal containing, as a constitutional unit, an organic molecule having a total of four or more specific heteroatoms in one molecule and an alkali metal salt was found to exhibit high ionic conductivity both at room temperature (25°C) and high temperature (80°C).

In contrast, in Comparative Example 1 in which a solid electrolyte was prepared using a molecular crystal containing, as a constitutional unit, an organic molecule having three or fewer specific heteroatoms and an alkali metal salt, the solid electrolyte melted at high temperature (80°C), and could not maintain a solid state under high temperatures.

The above results of evaluation have proven that a molecular crystal containing, as a constitutional unit, an organic molecule having four or more specific heteroatoms in one molecule and an alkali metal salt exhibits high ionic conductivity under high temperatures, while maintaining a solid state.

The present invention is not limited to the aforementioned embodiments. Needless to say, the present invention encompasses various modifications and those falling within the equivalents thereof, so long as they are not deviated from the gist of the present invention. Thus, it should be construed that, in view of the above teaching, those skilled in the art could conceive various combinations, modes, and further embodiments of a single element or a combination including the element or its equivalent, which also fall within the scope or concept of the present invention.

### Description of Reference Numerals

- 10: press mold

## Claims

1. A molecular crystal comprising:
an organic molecule having a total of four or more atoms of at least one species selected from the group consisting of a nitrogen atom, an oxygen atom, a sulfur atom, and a phosphorus atom; and
an alkali metal salt.

2. The molecular crystal according to claim 1, wherein the organic molecule has a total of four or more atoms of either or both of nitrogen and oxygen atoms.

3. The molecular crystal according to claim 1, wherein the organic molecule comprises a compound having two or more amide groups.

4. The molecular crystal according to claim 1, wherein the organic molecule comprises a compound having four or more oxygen atoms.

5. The molecular crystal according to claim 4, wherein the compound having four or more oxygen atoms is a crown ether, a polyol ester, or a lactide.

6. The molecular crystal according to claim 1, wherein an ionic conductivity at 25°C is 1.0 × 10⁻⁶ S/cm or higher.

7. The molecular crystal according to claim 1, wherein the alkali metal salt contains an imide-type alkali metal salt.

8. A solid electrolyte comprising the molecular crystal according to any one of claims 1 to 7.

9. A power storage device comprising the solid electrolyte according to claim 8.
